# EUROPÄISCHE PATENTSCHRIFT

(11) **EP 0 655 621 B1**
(45) Veröffentlichungstag und Bekanntmachung des Hinweises auf die Patenterteilung: **14.06.2006**
(21) Anmeldenummer: 94118541.5
(22) Anmeldetag: 25.11.1994
(51) Int. Cl.: G01N 21/90, B07C 5/34

(54) **Verfahren und Vorrichtung zur Unterscheidung zwischen produktspezifischen Inhaltsstoffen, sowie Kontaminationen von Fremdstoffen in Mehrweg-Getränkeflaschen**
Method and apparatus for differentiating between product-specific contents, as well as between contaminations due to foreign substances, in reusable beverage bottles
Procédé et dispositif pour différencier les constituants d'une boisson contenus dans des bouteilles récupérables, ainsi que les contaminations dues à des impuretés

(30) Priorität: 30.11.1993 DE 4340668
(43) Veröffentlichungstag der Anmeldung: 31.05.1995
(73) Patentinhaber: Krieg, Gunther, Prof.Dr.Ing., D-76227 Karlsruhe (DE)
(72) Erfinder: Dausch, Manfred Dipl.Ing., D-76831 Göcklingen (DE); Krieg, Gunther Prof.Dr.Ing., D-76227 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 557 814
- WO-A-88/00862
- DE-A- 4 200 971
- DE-A- 4 223 269
- US-A- 3 639 067
- US-A- 4 151 582
- US-A- 4 209 802
- US-A- 4 492 475
- US-A- 5 008 533
- US-A- 5 103 381

## Beschreibung

Gegenstand der Erfindung ist ein System zur Messung der Kontaminationen durch verschiedenste Stoffe, besonders solche mit niedrigen Partialdrücken wie Mineralöle, Speiseöle, Reinigungsmittel, Weichspüler, Waschmittel, Urin, usw. in Flaschen und Behältern, insbesondere auch in Getränke-Mehrweg-Kunststoffflaschen und -behältern.

Gemäß Patent-Anmeldung DE 42 00 971 A1 kann dies durch Durchstrahlung der Flasche bzw. des Behälters mit Licht innerhalb von Spektralbereichen, in denen die genannten Behälter transparent sind, durch Spektralanalyse erfolgen. Dabei werden u.a. die Absorptionsspektren als Fingerprints der Moleküle den einzelnen Molekülgruppen zugeordnet, sodaß eine mehr oder weniger selektive Erkennung und Unterscheidung zwischen Schadstoffen und Gutstoffen möglich ist.

Ein großes Problem bei der Anwendung dieses Verfahrens ergibt sich dadurch, daß einerseits der Weg der Lichtstrahlen durch die verschiedenen Brechungsindizes der Flüssigkeiten, als auch durch verschiedene Füllhöhen der Restflüssigkeiten, sowohl auch durch Toleranzen der Flaschenform, und durch Toleranzen der Prüfeinheit verändert werden, sodaß eine eindeutige Messung der spektralen Zusammensetzung mit großen Problemen verbunden ist. Hinzu kommt, daß die Aufgabenstellung die Messung kleinster Substanzmengen z.B. in Form von Flüssigkeitsfilmen, Flüssigkeitstropfen sowie von Einzelmolekülen in der Flaschenwand infolge Diffusion, sowie von Festkörperfilmen umfaßt.

Dementsprechend führt das in DE 42 00 971 A1 beschriebene Verfahren zu der Einschränkung in der technischen Anwendung, daß lediglich Stoffe bzw. Stoffgemische mit definiertem Brechungsindex, bei definierter Füllhöhe spektroskopisch erfaßt werden können. Bei Änderung dieser Parameter werden die dort angegebenen Strahlrichtungen so verändert, daß die spektroskopische Erfassung mit dem dort angegebenen optischen System nur mit großen Schwierigkeiten und Einschränkungen möglich ist. Ferner führt die dort angegebene vertikale Positionierung der Flaschen/Behälter zu einer Verteilung der Reststoffe über den Bodenbereich, sodaß die Stoffmenge am Durchstrahlort stark reduziert wird und Messungen nur oberhalb gewisser Mlndestmengen möglich sind.

Zur Lösung dieser Probleme werden in der vorliegenden Erfindung folgende Maßnahmen vorgeschlagen:
Zunächst wird im Unterschied zu dem in DE 42 00 971 A1 angegebenen parallelen Lichtstrahl, ein konvergenter Lichtstrahl verwendet. Ferner werden die durch die Flüssigkeit bzw. die Flaschenwandung auftretenden verschiedensten Strahlrichtungen mit einem speziellen Faser-Lichtsammler erfaßt und erst danach der Spektralauswertung zugeführt. Dieser Faser-Lichtsammler ist insbesondere dadurch gekennzeichnet, daß er die verschiedenen Strahlrichtungen erfaßt, alle Strahlrichtungen in eine Richtung umlenkt, und die aus verschiedenen Bereichen der Restsubstanz herrührenden Strahlen gleichmäßig und statistisch vermischt der Austrittsapertur zuführt, und damit sicherstellt, daß unabhängig von der Beleuchtungsrichtung immer das jeweilige Gesamtspektrum der Restsubstanzen vom nachgeschalteten Spektrometer erfaßt wird.

US-A-3 639 667 beschreibt einen breitwinkligen Faser-Lichtsammler.

Die Erfindung wird anhand der im folgenden dargestellten Figuren näher erläutert. Dabei erläutern im einzelnen:
**Fig.1** den prinzipiellen Aufbau der Gesamtanordnung.
**Fig.2a** eine Getränke-Mehrweg-Flasche im Bodenbereich und den für verschiedene
Flüssigkeiten spezifischen Strahlverlauf durch die Flasche.
**Fig.2b** die unterschiedlichen Austrittsstrahlen bei verschiedenen Restflüssigkeitsfüllständen.
**Fig.3** die Gesamtanordnung der optischen Strahlerzeugung und Strahlsammlung.
**Fig.4** die Eindüsung von Flüssigkeit in die Flasche/Behälter mit anschließender
Kippbewegung bei einer linearen Transportrichtung.
**Fig.5** die Sortierung der geprüften und identifizierten Flaschen/Behälter nach der Prüfvorrichtung.
**Fig.6** eine weitere Möglichkeit der Lichtsammlung.

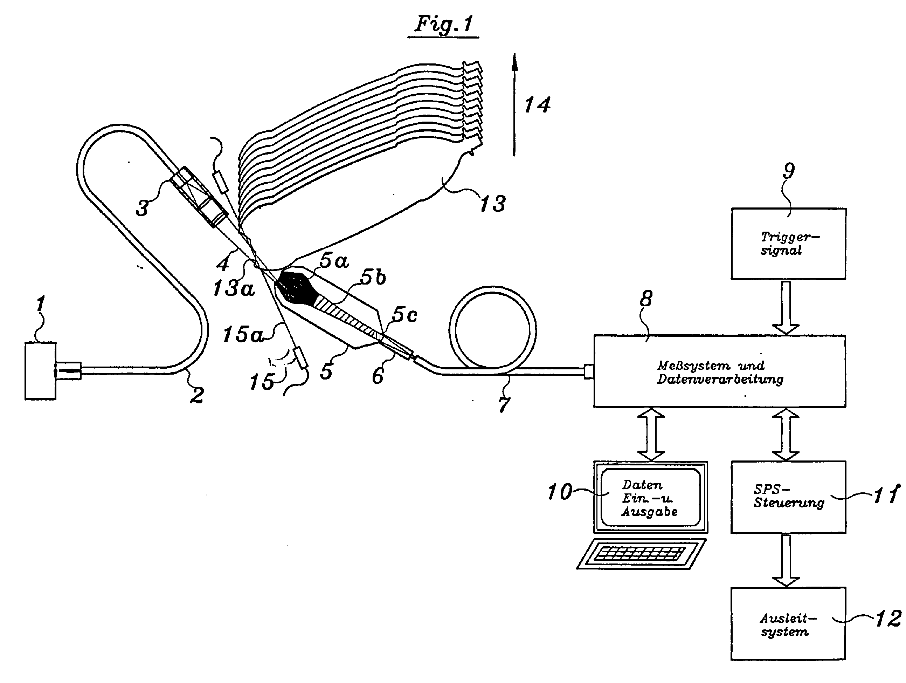

Das in **Fig.1** beschriebene Gesamtsystem besteht aus einer Lichtquelle (1), die vorzugsweise ein Xenonlampensystem ist. Das ausgesendete Licht wird über einen Lichtwellenleiter (2) und eine Strahlformoptik (3) in einen konvergenten Strahl (4) übergeführt, der an der Stelle (13a) die jeweilige Flasche in Bodennähe durchläuft und von einem Faser-Lichtsammler (5) erfaßt wird. Der Austrittsstrahl aus der Einkoppeloptik (6) gelangt über einen an das nachgeschaltete Spektrometer angepaßten 2.Lichtwellenleiter (7) auf das Meßsystem (8) mit integriertem Spektrometer und nachgeschalteter Datenverarbeitung. Das Spektrometer ist vorzugsweise ein mit holographischen Gitter ausgerüstetes Gitterspektrometer mit einem Sensorarray. Das Sensorarray weist zwischen 32 und 1024 Sensoren auf. Das von der Datenverarbeitung gelieferte Ergebnis, teilt die Flaschen in z.B. 3 Kategorien ein, nämlich in reine Mineralwasserflaschen, nicht kontaminierte Softdrinks und kontaminierte Flaschen. Der Durchlauf der Getränkeflaschen (13) in Richtung (14) wird über eine hochpräzise und ultraschnelle Laserlichtschranke (15, 15a) synchronisiert. Die Transportrichtung der Flaschen/Behälter kann eine geradlinige (14), oder jede andere, z.B. eine Rotationsbewegung sein. Die Synchronisation erfolgt über ein Triggersignal (9) an das Meßsystem. Die Ausleitung der untersuchten Flaschen in die oben genannten 3 Fraktionen erfolgt über das Ausleitsystem (12), welches von der Steuereinheit (11) beeinflußt wird. Die Daten-Ein.- und Ausgabe wird über das Terminal (10) abgewickelt.

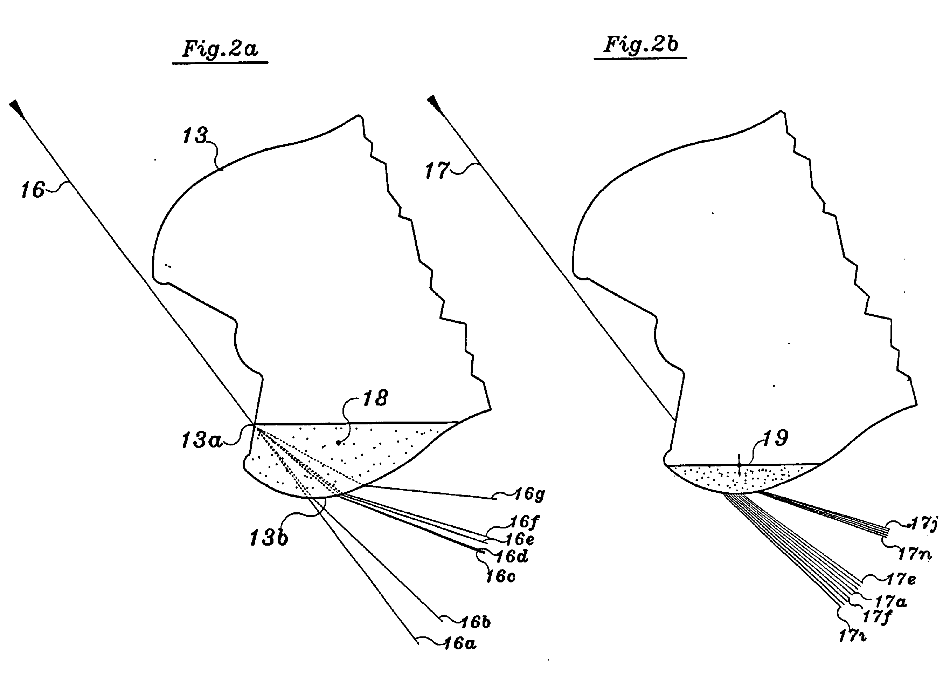

Gemäß **Fig.2a** wird der eintretende Lichtstrahl (16) durch die Restflüssigkeit (18) beim zweifachen Durchgang durch die Flaschenwandung (13a, 13b) zweifach gebrochen, wohingegen die Brechung durch die Flaschen-/Behälterwandung nur ein Parallelversatz hervorruft und außerdem als gegebene Größe immer bekannt ist und deshalb hier vernachlässigt werden kann. Falls sich keine Getränkeflasche im Lichtstrahl befindet verläuft der Strahl gemäß (16a), bei leerer Flasche entsprechend (16b). Bei mit verschiedenen Restflüssigkeiten bei konstantem Füllstand (18) verläuft der Strahl mit wachsendem Brechungsindex der Flüssigkeit gemäß (16c = Wasser), (16d = Urin), (16e = Cola), (16f = NaOCl Desinfektionsmittel) und (16g = Speiseöl).

In **Fig. 2b** sind die selben Verhältnisse bei ein und derselben Stoffzusammensetzung und verschiedenen Füllmengen (19) aufgezeigt. Das angegebene Beispiel zeigt eine Cola-Flüssigkeit bei 1 ml (17a) in Stufung von 1 ml (17a ... 17e, mit 17e = 5 ml), (17f = 6 ml) in Stufungen von 1ml (17f ... 17i, mit 17i = 9 ml) und (17j = 10 ml) in Stufungen von 1 ml bis (17j ... 17n, mit 17n = 14 ml).

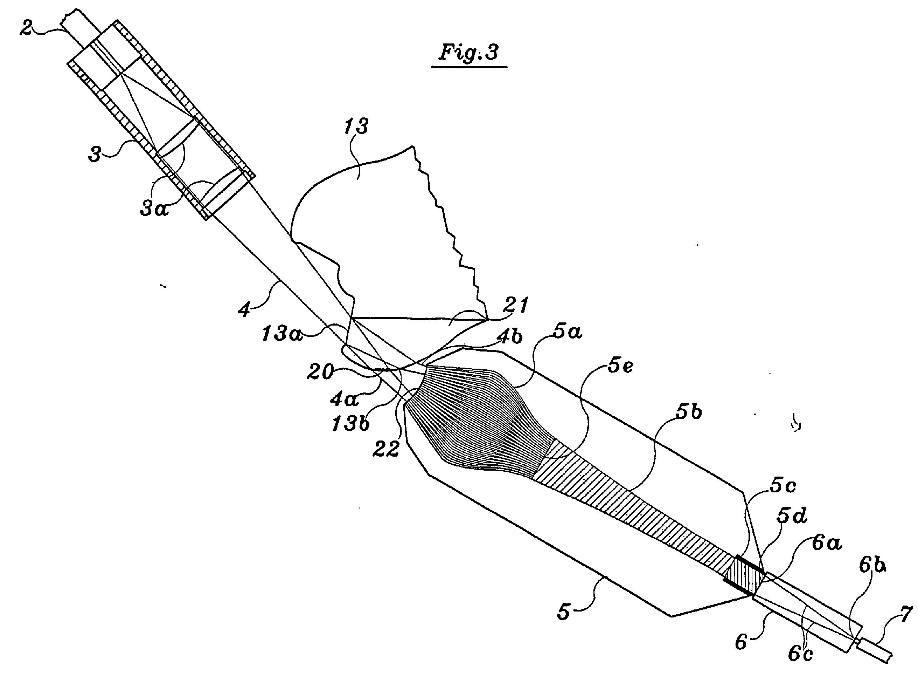

Die in **Fig.3** dargestellte detaillierte Darstellung der optischen Strahlführung durch den Flaschenbodenbereich (13a, 13b) mit an der Stelle 20 konzentrierten Reststoff bzw. der Flüssigkeit (21), erfolgt über den Lichtwellenleiter (2), den Lichtwellenleiterstecker (2a), ein achromatisches Linsensystem (3a) zur Farbkorrektur, zwecks Erzeugung eines konvergenten Strahls (4), der die Flasche (13) im Bereich (13a, 13b) durchläuft. Die aus der Flasche austretenden verschiedenen Strahlrichtungen (4a, 4b; 16a ... 16g, Fig.2a; 17a ... 17n, Fig.2b) werden von einem aus geordneten Lichtwellenleiterfasern (5a) bestehenden Faser-Lichtsammler (5) erfaßt, über einen optischen Mischer welcher die Fasern räumlich statistisch vermischt und von einem rechteckigen Eintrittsquerschnitt (5e) in einen kreisförmigen Austrittsquerschnitt (5d) überführt und danach der nachgeschalteten Einkoppeloptik (6) zugeführt. Eine Hülse (5c) positioniert die statistisch verteilten Fasern in fester Lage und ermöglicht an der Austrittsfläche (5d) ein Polieren der Stirnfläche, sodaß die Lichtverluste minimiert werden. Das Licht von der Ausgangsapertur (5d) wird über die Einkoppeloptik (6) mit Fokussierungswirkung über einen Lichtwellenleiter (7) auf das Spektrometer (8, Fig.1) gegeben. Die Einkoppeloptik (6), welche Im Kern aus Glas oder Quarzglas besteht, hat die Form eines Kreiskegelstumpfes (6, 6a, 6b, 6c) und ist an seinen runden, polierten Endflächen (6a, 6b) an die davor und danach positionierten Optiken (5, 7) flächenmäßig angepaßt. Eine 2.Schicht im Bereich der Mantelfläche (6c) besteht aus einem Glas- oder quarzglasdotiertem Material, um eine Totalreflexion an der Stelle (6c) der von der Eintrittsfläche (6a) herkommenden Eingangsstrahlen zu gewährleisten, und um die geeignete -an die nachgeschaltete Optik (7) angepaßte- Ausgangsapertur zu erreichen.

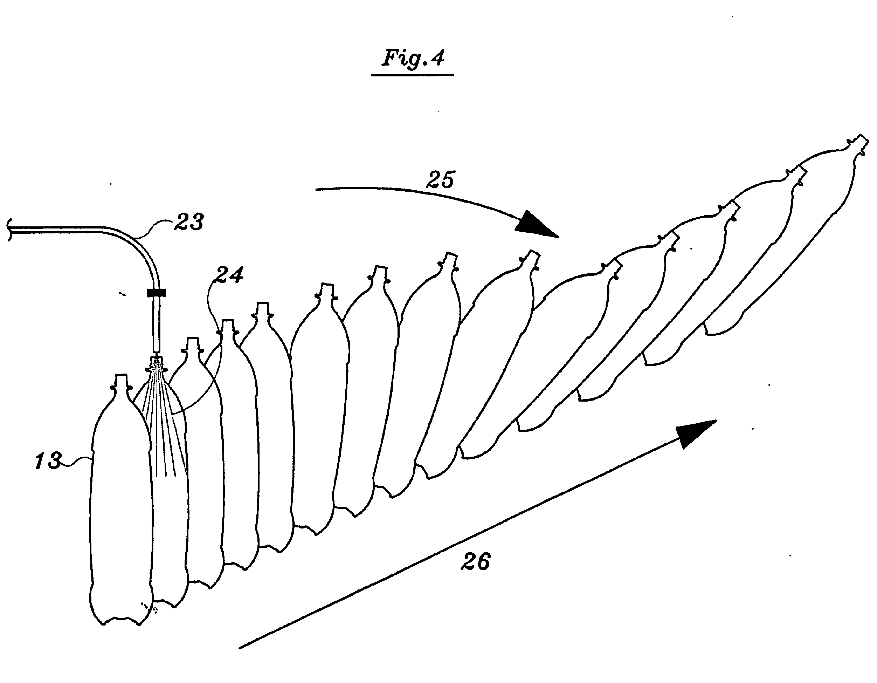

Gemäß **Fig.4** wird während der Linearbewegung in Richtung (26) in die Flaschen/Behälter (13) Flüssigkeitsmengen zwischen 2 %o und 2 % des Flaschen-/Behältervolumens eingedüst, um u.a. auch die eventuell eingetrockneten Feststoffe zu lösen. Die Eindüsung erfolgt über die Vorrichtung (23). Die Flüssigkeitsstrahlrichtung (24) ist so eingestellt, daß die Flüssigkeit im oberen Bereich der Flaschen-/Behälterinnenwand auftrifft, um so die eventuell an der Wandung befindlichen Eintrocknungen abzulösen und abzuspülen, damit sie sich dann im Bodenbereich sammeln können. Anschließend werden die Flaschen/Behälter in Richtung (25) bis zur optimalen Meßposition gekippt, um dann in dieser Lage vom danach kommenden Sensorsystem erfaßt werden zu können. Aus Versuchen wurde ermittelt, daß die optimale Kipplage gegenüber der Vertikalachse der Flasche/Behälter zwischen 55° und 75° liegt.

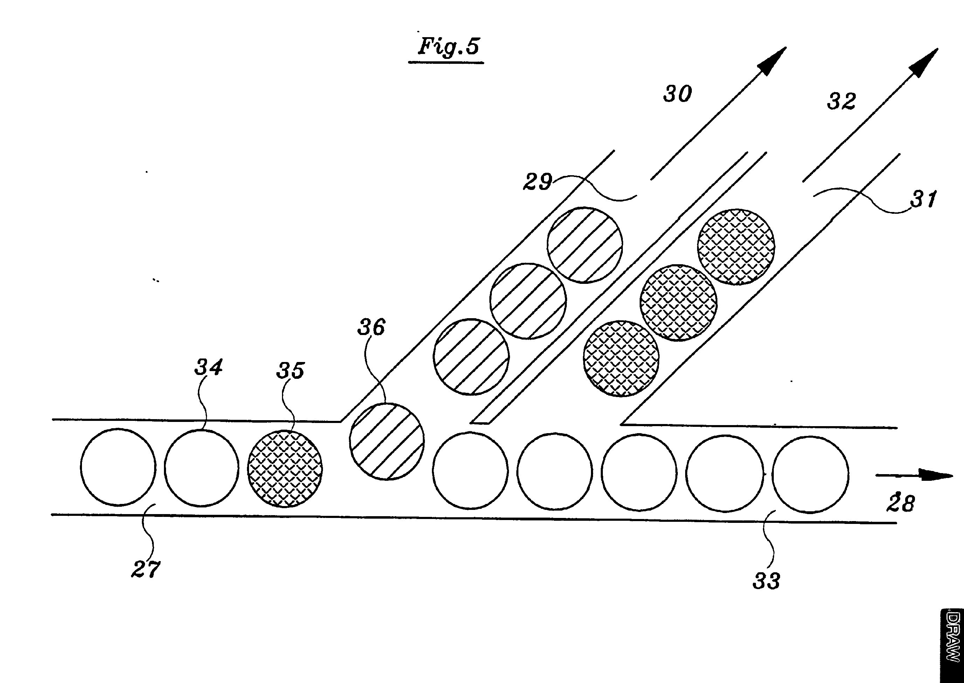

**Fig.5** zeigt, wie über das gemeinsame Transportband (27), die identifizierten Flaschen/Behälter (34, 35, 36) je nach Inhaltsstoffen in die drei Sortierungsrichtungen (28, 30 oder 32) geleitet werden. Dabei entsprechen die Flaschen/Behälter (34) auf Band (33) denen, die als Originalprodukt Mineralwasser enthielten, die auf Band (29) denen, die Softdrinks wie Orangenlimonade, Cola, usw. enthielten und auf Band (31) denen, die Kontaminationen verschiedenster Fremdstoffe enthalten.

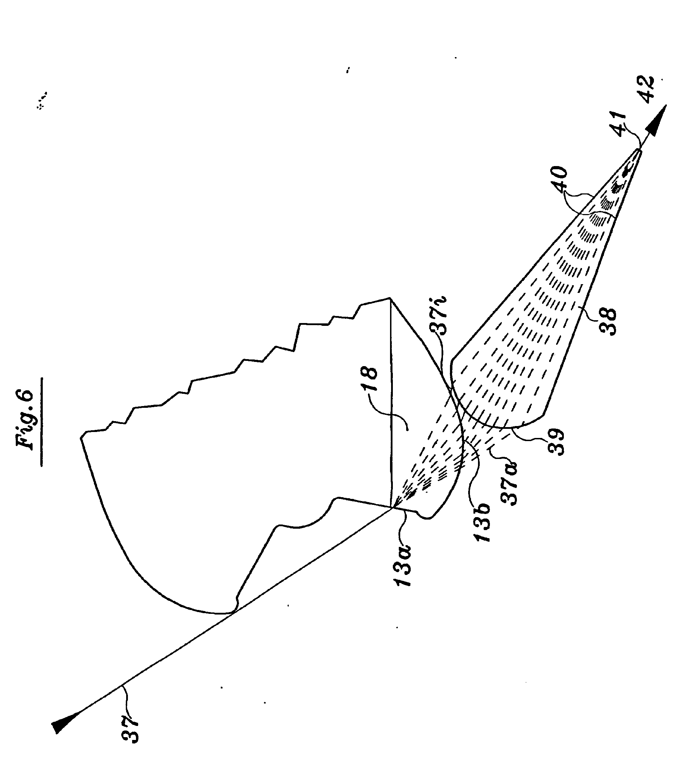

In **Fig.6** ist eine weitere Möglichkeit der Lichtsammlung (38, 39, 40, 41) der aus dem Flaschen-/Behälterbodenbereich austretenden Lichtstrahlen (37a ... 37i) aufgezeigt. Der Lichtstrahl (37) wird, wie schon in (Fig.2a) gezeigt, beim zweimaligen Durchgang durch die Flaschen-/Behälterwandung (13a, 13b) durch die verschiedenen Flüssigkeiten mit gleichem Füllstand (18) zweifach gebrochen, in die Strahlrichtungen (37a ... 371) abgelenkt und von einem aus Glas oder Quarzglas bestehendem Kegel-Lichtsammler (38) erfaßt und in Richtung (42) weitergeleitet. Die konkave Eintrittsfläche (39) hat eine solche Kontur, daß die Eintrittsstrahlen (37a ... 37i) in Richtung (42) gebrochen werden. Treten Strahlabweichungen durch Toleranzen der Flaschen/Behälter oder der Transportmechanik auf, können die Eintrittsstrahlen (37a ... 37i) dennoch über die Seitenflächen (40) -die durch Aufbringen eines geeigneten zweiten Materials oder durch Verspiegelung- durch Totalreflexion auf die senkrecht zur Längsachse und polierten Endfläche (41) weitergeleitet und damit vom nachfolgenden Lichtwellenleiterkabel (7, Fig.3) erfaßt werden. Als Strahleintrittsfläche (39) ist eine rechteckige, in Strahlablenkungsrichtung weiter ausgedehnte Fläche, oder auch eine linsenförmige, konvexe, runde möglich. Das letztgenannte System besitzt einen Körper -ausgenommen der Strahleintrittsfläche- analog einem Kreiskegelstumpf (40,41).

## Patentansprüche

1. Verfahren zur Messung von Kontaminationen in Form von Flüssigkeiten, Flüssigkeitsfilmen, Feststoffen und Feststoffilmen in Mehrweg-Flaschen/Behältern, insbesondere aus Kunststoff, unter Einsatz eines optischen Spektrometers, bei Durchstrahlung der Flaschen-/Behälterwandung insbesondere in der Lebensmittel- und Getränke Industrie, **dadurch gekennzeichnet, daß** die aus der Flaschenwandung austretende Strahlung unterschiedlicher Strahlrichtungen mittels eines Faser-Lichtsammlers bei welchem die Richtungen der Lichtwellenleiterfasern im wesentlichen den Richtungen der gebrochenen aus der Flaschenwand austretenden Strahlen entsprechen, in eine Richtung umgelenkt und anschließend auf das Spektrometer geleitet werden, worin die am Eintritt des Faser-Lichtsammlers inhomogene Intensitätsverteilung durch einen optischen Mischer in eine weitgehend homogene Intensitätsverteilung am Strahlaustrittsort übergeführt wird.

2. Verfahren nach Anspruch 1, **dadurch gekennzeichnet, daß** der Faser-Lichtsammler aus Lichtwellenleiterfasern besteht.

3. Verfahren nach Anspruch 1 bis 2, **dadurch gekennzeichnet, daß** die rechteckförmige Eintrittsapertur des Faser-Lichtsammlers konkav gekrümmt ist.

4. Verfahren nach Anspruch 1 bis 3, **dadurch gekennzeichnet, daß** die Krümmung der Eintrittsapertur des Faser-Lichtsammlers durch Aufeinanderschichtung räumlich versetzter, bandförmiger und an der Stirnseite rechteckförmiger Faserbündel erreicht wird.

5. Verfahren nach Anspruch 1 bis 4, **dadurch gekennzeichnet, daß** der Faser-Lichtsammler eine rechteckförmige Eintrittsapertur besitzt und die Austrittsapertur kreisförmig ist.

6. Verfahren nach Anspruch 1 bis 5, **dadurch gekennzeichnet, daß** beim Faser-Lichtsammler die Ausdehnung der Strahleintrittsfläche in Strahlablenkungsrichtung wesentlich größer ist, als die Ausdehnung in der dazu senkrechten Richtung.

7. Verfahren nach Anspruch 1 bis 6, **dadurch gekennzeichnet, daß** die Flaschen/Behälter vor der Messung bezüglich der Vertikalachse gekippt werden, und die Messung im Flüssigkeitsansammelbereich durchgeführt wird.

8. Verfahren nach Anspruch 1 bis 7, **dadurch gekennzeichnet, daß** der Kippwinkel der Flaschen/Behälter gegenüber der Vertikalen 55°...75° beträgt.

9. Verfahren nach Anspruch 1 bis 8, **dadurch gekennzeichnet, daß** zusätzlich Flüssigkeit in die Flaschen/Behälter eingespritzt wird.

10. Verfahren nach Anspruch 1 bis 9, **dadurch gekennzeichnet, daß** die in die Flaschen/Behälter eingespritzte Flüssigkeitsmenge etwa 2%o...2% des Behältervolumens beträgt.

11. Verfahren nach Anspruch 1 bis 10, **dadurch gekennzeichnet, daß** der Kern der Einkoppeloptik aus Quarzglas und die 2. Schicht im Bereich der Mantelfläche aus quarzglasdotiertem Material besteht.

12. Verfahren nach Anspruch 1 bis 11, **dadurch gekennzeichnet, daß** der Kern der Einkoppeloptik aus Glas und die 2. Schicht im Bereich der Mantelfläche aus glasdotiertem Material besteht.

13. Verfahren nach Anspruch 1 bis 12, **dadurch gekennzeichnet, daß** die Einkoppeloptik flächenmäßig an die davor- und danachgeschaltete Optik angepaßt ist.

14. Verfahren nach Anspruch 1 bis 13, **dadurch gekennzeichnet, daß** die 2.Schicht im Bereich der Mantelfläche bei der Einkoppeloptik so gewählt wird, daß die Ausgangsapertur an die danach folgende Optik angepaßt ist.

15. Verfahren nach Anspruch 1 und 7 bis 10, **dadurch gekennzeichnet, daß** die konvexe Strahleintrittsfläche des Kegel-Lichtsammlers so gekrümmt ist, daß die verschiedenen Strahlrichtungen aus der Flaschen-/Behälterwandung durch Lichtbrechung in Richtung der Lichtaustrittsfläche umgelenkt werden.

16. Verfahren nach Anspruch 15, **dadurch gekennzeichnet, daß** die durch Toleranzen und verschiedenen Flüssigkeitsfüllständen herrührenden Strahlabweichungen, durch Totalreflexion an der Mantelfläche des Kegel-Lichtsammlers in die Lichtaustrittsrichtung gelenkt werden.

17. Verfahren nach Anspruch 15 und 16, **dadurch gekennzeichnet, daß** die Strahleintrittsfläche des Kegel-Lichtsammlers eine runde und konvexe Fläche besitzt.

18. Verfahren nach Anspruch 15 bis 17, **dadurch gekennzeichnet, daß** die Strahleintrittsfläche des Kegel-Lichtsammlers eine rechteckförmige, in Strahlauslenkungsrichtung weiter ausgedehnte Fläche besitzt.

19. Vorrichtung zur Messung von Kontaminationen in Form von Flüssigkeiten, Flüssigkeitsfilmen, Feststoffen und Feststoffilmen in Mehrweg-Flaschen/Behältern (13), insbesondere aus Kunststoff, unter Einsatz eines optischen Spektrometers (8), bei Durchstrahlung der Flaschen-/Behälterwandung (13a, 13b) insbesondere in der Lebensmittel- und Getränke Industrie, **dadurch gekennzeichnet, daß** die aus der Flaschenwandung (13b) austretende Strahlung unterschiedlicher Strahlrichtungen (16a, 16g, 17a, 17n) mittels eines Faser-Lichtsammlers (5), bei welchem die Richtungen der Lichtwellenleiterfasern (5a) im wesentlichen den Richtungen der gebrochenen aus der Flaschenwandung austretenden Strahlen (16a, 16g, 17a, 17n) entsprechen, in eine Richtung umgelenkt und anschließend auf das Spektrometer geleitet werden, worin die am Eintritt des Faser-Lichtsammlers (5, 22) inhomogene Intensitätsverteilung durch einen optischen Mischer (5b) in eine weitgehend homogene Intensitätsverteilung am Strahlaustrittsort (5d) übergeführt wird.

20. Vorrichtung nach Anspruch 19, **dadurch gekennzeichnet, daß** der Faser-Lichtsammler (5) aus Lichtwellenleiterfasern (5a, 5b, 5c) besteht.

21. Vorrichtung nach Anspruch 19 und 20, **dadurch gekennzeichnet, daß** die rechteckförmige Eintrittsapertur (22) des Faser-Lichtsammlers (5) konkav gekrümmt ist.

22. Vorrichtung nach Anspruch 19 bis 21, **dadurch gekennzeichnet, daß** die Krümmung der Eintrittsapertur (22) des Faser-Lichtsammlers (5) durch Aufeinanderschichtung räumlich versetzter, bandförmiger und an der Stirnseite rechteckförmiger Faserbündel (5a) erreicht wird.

23. Vorrichtung nach Anspruch 19 bis 22, **dadurch gekennzeichnet, daß** der Faser-Lichtsammler (5) eine rechteckförmige Eintrittsapertur (22) besitzt und die Austrittsapertur (5d) kreisförmig ist.

24. Vorrichtung nach Anspruch 19 bis 23, **dadurch gekennzeichnet, daß** beim Faser-Lichtsammler (5) die Ausdehnung der Strahleintrittsfläche (22) in Strahlablenkungsrichtung wesentlich größer ist, als die Ausdehnung in der dazu senkrechten Richtung.

25. Vorrichtung nach Anspruch 19 bis 24, **dadurch gekennzeichnet, daß** die Flaschen/Behälter (13, Fig.4) vor der Messung bezüglich der Vertikalachse gekippt (25) werden, und die Messung im Flüsslgkeitsansammelbereich (20, Fig.3) durchgeführt wird.

26. Vorrichtung nach Anspruch 19 bis 25, **dadurch gekennzeichnet, daß** der Kippwinkel (25) der Flaschen/Behälter (13, Fig.4) gegenüber der Vertikalen 55°...75° beträgt.

27. Vorrichtung nach Anspruch 19 bis 26, **dadurch gekennzeichnet, daß** zusätzlich Flüssigkeit (23,24) in die Flaschen/Behälter (13, Fig.4) eingespritzt wird.

28. Vorrichtung nach Anspruch 19 bis 27, **dadurch gekennzeichnet, daß** die in die Flaschen/Behälter (13, Fig.4) eingespritzte Flüssigkeitsmenge etwa 2‰...2% des Behältervolumens beträgt.

29. Vorrichtung nach Anspruch 19 bis 28, **dadurch gekennzeichnet, daß** der Kern (6a, 6b) der Einkoppeloptik (6) aus Quarzglas und die 2. Schicht im Bereich der Mantelfläche (6c) aus quarzglasdotiertem Material besteht.

30. Vorrichtung nach Anspruch 19 bis 29, **dadurch gekennzeichnet, daß** der Kern der Einkoppeloptik (6a, 6b) aus Glas und die 2. Schicht im Bereich der Mantelfläche (6c) aus glasdotiertem Material besteht.

31. Vorrichtung nach Anspruch 19 bis 30, **dadurch gekennzeichnet, daß** die Einkoppeloptik (6) flächenmäßig (6a, 6b) an die davor- und danachgeschaltete Optik (5, 7) angepaßt ist.

32. Vorrichtung nach Anspruch 19 bis 31, **dadurch gekennzeichnet, daß** die 2.Schicht im Bereich der Mantelfläche (6c) bei der Einkoppeloptik so gewählt wird, daß die Ausgangsapertur (6b) an die danach folgende Optik (7) angepaßt ist.

33. Vorrichtung nach Anspruch 19 bis 32, **dadurch gekennzeichnet, daß** die konvexe Strahleintrittsfläche (39) des Kegel-Lichtsammlers (38) so gekrümmt ist, daß die verschiedenen Strahlrichtungen aus der Flaschen-/Behälterwandung (37a ... 37i, Fig.6) durch Lichtbrechung in Richtung der Lichtaustrittsfläche (42) umgelenkt werden.

34. Vorrichtung nach Anspruch 33, **dadurch gekennzeichnet, daß** die durch Toleranzen und verschiedenen Flüssigkeitsfüllständen herrührenden Strahlabweichungen, durch Totalreflexion an der Mantelfläche (40) des Kegel-Lichtsammlers (38) in die Lichtaustrittsrichtung (42) reflektiert werden.

35. Vorrichtung nach Anspruch 33 und 34, **dadurch gekennzeichnet, daß** die Strahleintrittsfläche (39) des Kegel-Lichtsammlers (38) eine runde und konvexe Fläche besitzt.

36. Vorrichtung nach Anspruch 33 bis 35, **dadurch gekennzeichnet, daß** die Strahleintrittsfläche (39) des Kegel-Lichtsammlers (38) eine rechteckförmige, in Strahlauslenkungsrichtung weiter ausgedehnte Fläche besitzt.

## Claims

1. Method for measuring contaminations in the form of liquids, liquid films, solids and solid films in reusable bottles/containers, particularly made from plastic, using an optical spectrometer, on radiating through the bottle/container wall, particularly in the food and beverages industry, **characterized in that** the radiation from the bottle wall with different radiation directions is deflected by means of a fibre light collector, in which the directions of the light waveguide fibres substantially correspond to the directions of the refracted beams leaving the bottle wall, into a single direction and are subsequently guided to the spectrometer, in which the inhomogeneous intensity distribution at the entrance of the fibre light collector is transformed by an optical mixer into a substantially homogeneous intensity distribution at the beam exit point.

2. Method according to claim 1, **characterized in that** the fibre light collector comprises light waveguide fibres.

3. Method according to claims 1 and 2, **characterized in that** the rectangular entrance aperture of the fibre light collector is concavely curved.

4. Method according to claim 1 to 3, **characterized in that** the curvature of the entrance aperture of the fibre light collector is brought about by the stacking of spatially displaced, ribbonlike fibre bundles which are rectangular at the end face.

5. Method according to claims 1 to 4, **characterized in that** the fibre light collector has a rectangular entrance aperture and the exit aperture is circular.

6. Method according to claims 1 to 5, **characterized in that** in the case of the fibre light collector the extension of the beam entrance face in the beam deflecting direction is much larger than the extension in the direction perpendicular thereto.

7. Method according to claims 1 to 6, **characterized in that** the bottles/containers are tilted with respect to the vertical axis prior to the measurement, which is carried out in the liquid collecting area.

8. Method according to claims 1 to 7, **characterized in that** the tilt angle of the bottles/containers relative to the vertical is 55 to 75°.

9. Method according to claims 1 to 8, **characterized in that** additional liquid is injected into the bottles/containers.

10. Method according to claims 1 to 9, **characterized in that** the liquid quantity injected into the bottles/containers represents approximately 0.2 to 2% of the container volume.

11. Method according to claims 1 to 10, **characterized in that** the core of the coupling in optics is made from quartz glass and the second coating in the vicinity of the circumferential surface from quartz glass-doped material.

12. Method according to claims 1 to 11, **characterized in that** the core of the coupling in optics is made from glass and the second coating in the vicinity of the circumferential surface from glass-doped material.

13. Method according to claims 1 to 12, **characterized in that** the area of the coupling in optics is adapted to the upstream and downstream optics.

14. Method according to claims 1 to 13, **characterized in that** the second coating in the vicinity of the circumferential surface of the coupling in optics is chosen in such a way that the output aperture is adapted to the following optics.

15. Method according to claims 1 and 7 to 10, **characterized in that** the convex beam entrance face of the conical light collector is curved in such a way that the different beam directions from the bottle/container wall are deflected in the light exit face direction by light refraction.

16. Method according to claim 15, **characterized in that** the beam deviations resulting from tolerances and different liquid fill levels are deflected in the light exit direction by total reflection on the circumferential surface of the conical light collector.

17. Method according to claims 15 and 16, **characterized in that** the beam entrance face of the conical light collector has a circular, convex surface.

18. Method according to claims 15 to 17, **characterized in that** the beam entrance face of the conical light collector has a rectangular surface further extended in the beam deflection direction.

19. Apparatus for measuring contaminations in the form of liquids, liquid films, solids and solid films in reusable bottles/containers (13), particularly made from plastic, using an optical spectrometer (8), on radiating through the bottle/container wall (13a, 13b), particularly in the food and beverages industry, **characterized in that** the radiation from the bottle wall (13b) with different radiation directions (16a, 16g, 17a, 17n) is deflected by means of a fibre light collector (5), in which the directions of the light waveguide fibres substantially correspond to the directions of the refracted beams (16a, 16g, 17a, 17n) leaving the bottle wall, into a single direction and are subsequently guided to the spectrometer, in which the inhomogeneous intensity distribution at the entrance of the fibre light collector (5, 22) is transformed by an optical mixer (5b) into a substantially homogeneous intensity distribution at the beam exit point (5d).

20. Apparatus according to claim 19, **characterized in that** the fibre light collector (5) comprises light waveguide fibres (5a, 5b, 5c).

21. Apparatus according to claims 19 and 20, **characterized in that** the rectangular entrance aperture (22) of the fibre light collector (5) is concavely curved.

22. Apparatus according to claims 19 to 21, **characterized in that** the curvature of the entrance aperture (22) of the fibre light collector (5) is brought about by stacking spatially displaced, ribbonlike fibre bundles (5a) which are rectangular at the end face.

23. Apparatus according to claims 19 to 22, **characterized in that** the fibre light collector (5) has a rectangular entrance aperture (22) and the exit aperture (5d) is circular.

24. Apparatus according to claims 19 to 23, **characterized in that** in the case of the fibre light collector (5) the extension of the beam entrance face (22) in the beam deflection direction is much larger than the extension in the direction perpendicular thereto.

25. Apparatus according to claims 19 to 24, **characterized in that** the bottles/containers (13, fig. 4) are tilted (25) relative to the vertical axis prior to the measurement which is performed in the liquid collecting area (20, fig. 3).

26. Apparatus according to claims 19 to 25, **characterized in that** the tilt angle (25) of the bottles/containers (13, fig. 4) is 55 to 75° relative to the vertical.

27. Apparatus according to claims 19 to 26, **characterized in that** additional liquid (23, 24) is injected into the bottles/containers (13, fig. 4).

28. Apparatus according to claims 19 to 27, **characterized in that** the liquid quantity injected to the bottles/containers (13, fig. 4) is approximately 0.2 to 2% of the container volume.

29. Apparatus according to claims 19 to 28, **characterized in that** the core (6a, 6b) of the coupling in optics (6) is made from quartz glass and the second coating in the vicinity of the circumferential surface (6c) from quartz glass-doped material.

30. Apparatus according to claims 19 to 29, **characterized in that** the core of the coupling in optics (6a, 6b) is made from glass and the second coating in the vicinity of the circumferential surface (6c) from glass-doped material.

31. Apparatus according to claims 19 to 30, **characterized in that** the area (6a, 6b) of the coupling in optics (6) is adapted to the upstream and downstream optics (5, 7).

32. Apparatus according to claims 19 to 31, **characterized in that** the second coating in the vicinity of the circumferential surface (6c) of the coupling in optics is chosen in such a way that the exit aperture (6b) is adapted to the following optics (7).

33. Apparatus according to claims 19 to 32, **characterized in that** the beam entrance face (39) of the conical light collector (38) is curved in such a way that the different beam directions from the bottle/container wall (37a...37i, fig. 6) are deflected in the direction of the light exit face (42) by light refraction.

34. Apparatus according to claim 33, **characterized in that** the beam deviations caused by tolerances and different liquid fill levels are reflected in the light exit direction (42) by total reflection on the circumferential surface (40) of the conical light collector (38).

35. Apparatus according to claims 33 and 34, **characterized in that** the beam entrance face (39) of the conical light collector (38) has a circular, convex surface.

36. Apparatus-according to claims 33 to 35, **characterized in that** the beam entrance face (39) of the conical light collector (38) has a rectangular surface further extended in the beam deflection direction.

## Revendications

1. Procédé de mesure de contaminations sous la forme de liquides, films liquides, matières solides et films de matières solides dans les bouteilles ou contenants consignés, en particulier en matière plastique, en utilisant un spectromètre optique, par irradiation de la paroi de la bouteille ou du contenant, en particulier dans l'industrie des denrées alimentaires et des boissons, **caractérisé en ce que** la radiation omnidirectionnelle émanant de la paroi de la bouteille peut être déviée dans une direction et ensuite dirigée sur le spectromètre au moyen d'un collecteur de lumière à fibres optiques dans lequel les directions des fibres guidant les ondes lumineuses correspondent sensiblement aux directions des rayons réfractés sortant de la paroi de la bouteille, la distribution d'intensité, non homogène à l'entrée du collecteur de lumière à fibres optiques, étant transformée par un mélangeur optique en une distribution d'intensité largement homogène à l'endroit de sortie des rayons.

2. Procédé selon la revendication 1, **caractérisé en ce que** le collecteur de lumière à fibres optiques est constitué de fibres guidant des ondes lumineuses.

3. Procédé selon la revendication 1 à 2, **caractérisé en ce que** l'ouverture d'entrée de forme rectangulaire du collecteur de lumière à fibres optiques est incurvée concave.

4. Procédé selon la revendication 1 à 3, **caractérisé en ce que** la courbure de l'ouverture d'entrée du collecteur de lumière à fibres optiques est obtenue par l'étagement de faisceaux de fibres décalés dans l'espace, affectant la forme de bandes et rectangulaires au niveau de leurs faces frontales.

5. Procédé selon la revendication 1 à 4, **caractérisé en ce que** le collecteur de lumière à fibres optiques présente une ouverture d'entrée de forme rectangulaire et **en ce que** l'ouverture de sortie est circulaire.

6. Procédé selon la revendication 1 à 5, **caractérisé en ce que** dans le collecteur de lumière à fibres optiques, l'extension de la surface d'entrée des rayons dans le sens de la déflexion est sensiblement supérieure à l'extension dans la direction qui lui est perpendiculaire.

7. Procédé selon la revendication 1 à 6, **caractérisé en ce qu'**avant la mesure, on incline les bouteilles ou les contenants par rapport à l'axe vertical, et on réalise la mesure dans la zone de rassemblement du liquide.

8. Procédé selon la revendication 1 à 7, **caractérisé en ce que** l'angle d'inclinaison des bouteilles ou des contenants par rapport à la verticale est de 55° à 75°.

9. Procédé selon la revendication 1 à 8, **caractérisé en ce que** l'on injecte dans les bouteilles ou les contenants du liquide supplémentaire.

10. Procédé selon la revendication 1 à 9, **caractérisé en ce que** la quantité de liquide injectée dans les bouteilles ou les contenants correspond à environ 2‰ à 2% du volume du contenant.

11. Procédé selon la revendication 1 à 10, **caractérisé en ce que** le noyau de l'optique d'injection est en verre de quartz et la deuxième couche dans la zone de la surface enveloppe en matériau dopé avec du verre de quartz.

12. Procédé selon la revendication 1 à 11, **caractérisé en ce que** le noyau de l'optique d'injection est en verre et la deuxième couche dans la zone de la surface enveloppe en matériau dopé avec du verre.

13. Procédé selon la revendication 1 à 12, **caractérisé en ce que** l'optique d'injection est adaptée du point de vue de la surface à l'optique montée en amont et en aval.

14. Procédé selon la revendication 1 à 13, **caractérisé en ce que** la deuxième couche dans la zone de la surface enveloppe est choisie dans le cas de l'optique d'injection de telle façon que l'ouverture de sortie est adaptée à l'optique suivante.

15. Procédé selon la revendication 1 et 7 à 10, **caractérisé en ce que** la surface convexe d'entrée des rayons du collecteur conique de lumière est incurvée de telle façon que les différentes directions de rayonnement émanant de la paroi de la bouteille ou du contenant sont déviées par réfraction en direction de la surface de sortie de la lumière.

16. Procédé selon la revendication 15, **caractérisé en ce que** les différences de rayonnement provenant de tolérances et de niveaux de remplissage en liquide variable, sont déviées en direction de la sortie de la lumière par réflexion totale sur la surface enveloppe du collecteur conique de lumière.

17. Procédé selon la revendication 15 et 16, **caractérisé en ce que** la surface d'entrée du rayonnement du collecteur conique de lumière présente une surface ronde et convexe.

18. Procédé selon la revendication 15 à 17, **caractérisé en ce que** la surface d'entrée du rayonnement du collecteur conique de lumière présente une surface de forme rectangulaire, plus étendue en direction de la déviation du rayonnement.

19. Dispositif de mesure de contaminations sous la forme de liquides, films de liquides, matières solides et films de matières solides dans des bouteilles ou des contenants (13) consignés, en particulier en matière plastique, en utilisant un spectromètre optique (8), par irradiation de la paroi (13a, 13b) de la bouteille ou du contenant, en particulier dans l'industrie des denrées alimentaires et des boissons, **caractérisé en ce que** la radiation omnidirectionnelle (16a, 16g, 17a, 17n) émanant de la paroi (13b) de la bouteille peut être déviée dans une direction et ensuite dirigée sur le spectromètre au moyen d'un collecteur de lumière à fibres optiques (5) dans lequel les directions des fibres guidant les ondes lumineuses (5a) correspondent sensiblement aux directions des rayons réfractés (16a, 16g, 17a, 17n) sortant de la paroi de la bouteille, la distribution d'intensité, non homogène à l'entrée du collecteur de lumière à fibres optiques (5,22), étant transformée par un mélangeur optique (5b) en une distribution d'intensité largement homogène à l'endroit de sortie du rayonnement (5d).

20. Dispositif selon la revendication 19, **caractérisé en ce que** le collecteur de lumière à fibres optiques (5) est composé de fibres de guidage d'ondes lumineuses (5a, 5b, 5c).

21. Dispositif selon la revendication 19 et 20, **caractérisé en ce que** l'ouverture d'entrée (22) de forme rectangulaire du collecteur de lumière à fibres optiques (5) est incurvée concave.

22. Dispositif selon la revendication 19 à 21, **caractérisé en ce que** la courbure de l'ouverture d'entrée (22) de forme rectangulaire du collecteur de lumière à fibres optiques (5) est obtenue par superposition de faisceaux de fibres (5a) décalés dans l'espace, affectant la forme de bandes et rectangulaires au niveau de leurs faces frontales.

23. Dispositif selon la revendication 19 à 22, **caractérisé en ce que** le collecteur de lumière à fibres optiques (5) présente une ouverture d'entrée (22) de forme rectangulaire et **en ce que** l'ouverture de sortie (5d) est circulaire.

24. Dispositif selon la revendication 19 à 23, **caractérisé en ce que** dans le collecteur de lumière à fibres optiques (5), l'extension de la surface d'entrée (22) des rayons dans le sens de la déflexion est sensiblement supérieure à l'extension dans la direction qui lui est perpendiculaire.

25. Dispositif selon la revendication 19 à 24, **caractérisé en ce qu'**avant la mesure, on incline (25) les bouteilles ou les contenants (13, fig.4) par rapport à l'axe vertical, et on réalise la mesure dans la zone de rassemblement du liquide (20, fig.3).

26. Dispositif selon la revendication 19 à 25, **caractérisé en ce que** l'angle d'inclinaison (25) des bouteilles ou des contenants (13, fig.4) par rapport à la verticale est de 55° à 75°.

27. Dispositif selon la revendication 19 à 26, **caractérisé en ce que** l'on injecte dans les bouteilles ou les contenants (13, fig.4) du liquide (23,24) supplémentaire.

28. Dispositif selon la revendication 19 à 27, **caractérisé en ce que** la quantité de liquide injectée dans les bouteilles ou les contenants (13, fig.4) correspond à environ 2‰ à 2% du volume du contenant.

29. Dispositif selon la revendication 19 à 28, **caractérisé en ce que** le noyau (6a, 6b) de l'optique d'injection (6) est en verre de quartz et la deuxième couche dans la zone de la surface enveloppe (6c) en matériau dopé avec du verre de quartz.

30. Dispositif selon la revendication 19 à 29, **caractérisé en ce que** le noyau de l'optique d'injection (6a, 6b) est en verre et la deuxième couche dans la zone de la surface enveloppe (6c) en matériau dopé avec du verre.

31. Dispositif selon la revendication 19 à 30, **caractérisé en ce que** l'optique d'injection (6) est adaptée du point de vue de la surface (6a,6b) à l'optique (5,7) montée en amont et en aval.

32. Dispositif selon la revendication 19 à 31, **caractérisé en ce que** la deuxième couche dans la zone de la surface enveloppe (6c) est choisie dans le cas de l'optique d'injection de telle façon que l'ouverture de sortie (6b) est adaptée à l'optique suivante (7).

33. Dispositif selon la revendication 19 à 32, **caractérisé en ce que** la surface convexe (39) d'entrée des rayons du collecteur conique de lumière (38) est incurvée de telle façon que les différentes directions de rayonnement émanant de la paroi de la bouteille ou du contenant (37a à 37i, fig.6) sont déviées par réfraction en direction de la surface (42) de sortie de la lumière.

34. Dispositif selon la revendication 33, **caractérisé en ce que** les différences de rayonnement provenant de tolérances et de niveaux de remplissage en liquide variable, sont déviées en direction de la sortie de la lumière (42) par réflexion totale sur la surface enveloppe (40) du collecteur conique de lumière (38).

35. Dispositif selon la revendication 33 et 34, **caractérisé en ce que** la surface d'entrée (39) du rayonnement du collecteur conique de lumière (38) présente une surface ronde et convexe.

36. Dispositif selon la revendication 33 à 35, **caractérisé en ce que** la surface d'entrée (39) du rayonnement du collecteur conique de lumière (38) présente une surface de forme rectangulaire, plus étendue en direction de la déviation du rayonnement.
